# FASCICULE DE BREVET EUROPEEN

(11) **EP 1 095 035 B1**
(45) Date de publication et mention de la délivrance du brevet: **17.03.2010**
(21) Numéro de dépôt: 99929431.7
(22) Date de dépôt: 08.07.1999
(51) Int. Cl.: C07D 401/04, C07D 213/51

(54) **PROCEDE DE PREPARATION DU 4-(3-PYRIDINYL)-1H-IMIDAZOLE ET LES INTERMEDIAIRES MIS EN OEUVRE**
VERFAHREN ZUR HERSTELLUNG VOM 4-(3-PYRIDINYL)-1H-IMIDAZOL UND DIE ZUGEHÖRIGEN ZWISCHENPRODUKTE
METHOD FOR PREPARING 4-(3-PYRIDINYL)-1H-IMIDAZOLE AND THE INTERMEDIATES USED

(30) Priorité: 09.07.1998 FR 9808796
(43) Date de publication de la demande: 02.05.2001
(73) Titulaire: Aventis Pharma S.A., 92160 Antony (FR)
(72) Inventeur: BOUCHET, Rapha¬l, 93310 Le Pré Saint Gervais (FR); LAGOUARDAT, Jacques, F-93160 Noisy le Grand (FR); SCHOLL, Jacques, F-93230 Romainville (FR)
(86) Numéro de dépôt international: PCT/FR1999/001649
(87) Numéro de publication internationale: WO 2000/002875

(56) Documents cités:
- FR-A- 1 259 360
- US-A- 4 302 464
- J.L. LAMATTINA ET AL.: "Use of the Neber rearrangement for the synthesis of alpha-amino acetals" SYNTHESIS., no. 4, 1980, pages 329-330, XP002095693 STUTTGART DE

## Description

La présente invention concerne un procédé de préparation du 4-(3-pyridinyl)-1H-imidazole, de certains de ses dérivés ainsi que les produits intermédiaires mis en oeuvre.

L'invention a pour objet un procédé de préparation de composés de formule (I) 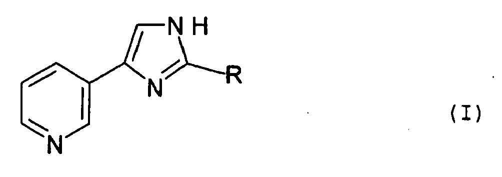 dans laquelle R représente un atome d'hydrogène ou un radical alkyle renfermant jusqu'à 8 atomes de carbone **caractérisé en ce que** l'on soumet un composé de formule (A) :

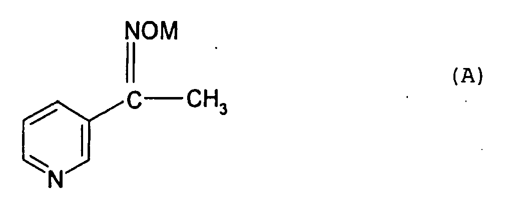

OM représentant un radical hydroxyle bloqué sous forme d'un bon groupe partant, à l'action d'un alcool et d'un alcoolate alcalin, alc₁OH/alc₂OL, alc₁ et alc₂, identiques ou différents, représentant un radical alkyle renfermant jusqu'à 4 atomes de carbone et L représentant un atome de sodium ou de potassium, pour obtenir la 3-(2H-azirin-3-yl)pyridine de formule B : 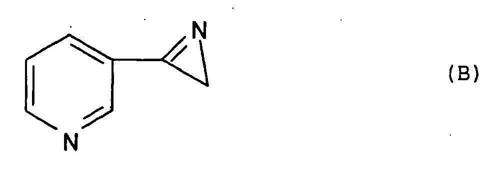 dans laquelle R conserve sa signification précédente que l'on soumet à une cyclisation pour obtenir le produit de formule (I) recherché.

Lorsque R représente un radical alkyle, il s'agit par exemple du radical méthyle, éthyle, n-propyle, isopropyle, n-butyle, isobutyle ou tertbutyle
Alc représente par exemple un radical méthyle, éthyle ou n-propyle.

Dans un mode de réalisation préféré, la réaction entre les composés (II) et (III) a lieu au sein d'un mélange d'alcool et de formamide. Comme alcool on peut citer le méthanol, l'éthanol ou le propanol.

La cyclisation du composé de formule (IV) a lieu au sein du formamide.

L'invention a plus particulièrement pour objet la préparation du composé de formule (I) dans lequel R est un atome d'hydrogène.

L'invention a plus particulièrement pour objet un procédé **caractérisé en ce que** alc représente un radical méthyle.

La cyclisation a lieu de préférence par chauffage.

Le 4-(3-pyridinyle)-1H imidazole est un produit connu décrit par exemple dans J. Chem.-Soc.-753-5 1938 qui peut-être utilisé pour préparer des produits pharmaceutiques (cf. EP 680967).

L'invention a également pour objet un procédé caractérisé en ce que le produit de formule (II) est préparé par action d'un alcool et d'un alcoolate alcalin, alc₁OH/alc₂OM, alc₁ et alc₂ identiques ou différents représentant un radical alkyle renfermant jusqu'à 4 atomes de carbone et M représentant un atome de sodium ou de potassium sur un composé de formule (A),

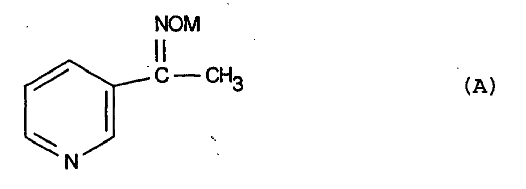

Dans un mode de réalisation référé :
- M est un radical tosyle,
- alc₁ et alc₂ représentent le même radical alkyle,
- on utilise le méthanol en présence de méthylate de sodium,
- l'acide est l'acide oxalique,
- la réaction entre les composés (II) et (III) a lieu au sein d'un mélange d'alcool, déjà présent dans le milieu réactionnel, et de formamide,
- la cyclisation du composé de formule (IV) a lieu au sein du formamide.

L'invention a plus particulièrement pour objet la préparation du composé de formule (I) telle que définie précédemment, caractérisée en ce que l'on utilise un composé de formule III dans laquelle R est un atome d'hydrogène.

L'invention a tout spécialement pour objet un procédé dans lequel il n'y a pas isolement de l'intermédiaire de formule (IV).

L'invention a plus particulièrement pour objet un procédé **caractérisé en ce que** l'on soumet la O-[(4-méthylphényl) sulfonyl] oxime de 1-(3-pyridinyl)-éthanone à l'action du méthanol et du méthylate de sodium pour obtenir la 3-(2H-azirin-3-yl)-pyridine que l'on soumet, sans l'isoler du milieu réactionnel, à l'action de l'acide oxalique pour obtenir la β,β-diméthoxy-2-(3-pyridinyl)éthyl]-amine que l'on soumet, sans l'isoler du milieu réactionnel, à l'action du formamide pour obtenir le N-[β,β-diméthoxy-2-(3-pyridinyl) éthyl]-formamide que l'on soumet, sans l'isoler du milieu réactionnel, à une cyclisation par chauffage pour obtenir le produit recherché.

Le document J.L. Lamattina et al, Synthesis n°4, 1980, p.329-330 décrit l'utilisation d'un réarrangement de Neber dans la synthèse d'α-amino acétals au départ de dérivés d'oximes, lesquels sont mis en oeuvre dans le brevet US 4.302.464 pour préparer des 4-(4-imidazolyl) pyridines par action d'un réactif de type imidate.

Par ailleurs, la demande japonaise publiée No.2-145572 décrit des composés de type N-[β,β-dialkoxy-2-(3-pyridinyl)éthyl]-amide.

La présente invention réalise la synthèse des composés de formule (I) en mettant notamment en oeuvre un réactif de type amide et dans des conditions particulièrement avantageuses, le cas échéant sans l'isolement d'aucun intermédiaire.

Le 4-(3-pyridinyl)-1H imidazole est un produit connu décrit par exemple dans J. Chem.-Soc.-753-5 (1938), qui peut-être utilisé pour préparer des produits pharmaceutiques comme décrit dans le brevet européen EP 680967.

L'exemple suivant illustre l'invention sans toutefois la limiter.

### Préparation : β,β-diméthoxy-3-pyridine éthanamine

### Stade A : 3-[2H-azirin-3-yl)-pyridine

On introduit à 20°C sous atmosphère d'azote 100 g de 0-[(4-méthylphényl)sulfonyl]-oxime de 1-(3-pyridinyl)-éthanone dans une solution renfermant 400 ml de méthanol anhydre et 23,45 g de méthylate de sodium. On agite 2 heures à 20∼22°C et obtient une suspension renfermant le produit recherché. Stade B : β,β-diméthoxy-3-pyridine éthanamine.

On refroidit à 0°C la suspension obtenue précédemment et ajoute 31,03 g d'acide oxalique. On agite pendant 15 minutes à 0°/+5°C. On obtient une suspension que l'on utilisé telle quelle dans l'exemple 1. Après isolement et purification, on a obtenu le β,β-diméthoxy-3-pyridine éthanamine.
RMN CDCl₃ ppm
1,03(m large) NH₂ supposé mobile
3,02(s) 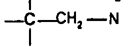 3,22(s) 6H 2 OCH₃
7,33(ddl, J=5 et 9) H₅
7,80(dt, J=9 et 2) H₄
8,59(dd, J=5 et 2) H₆
8,73(dd, J=2 et 1) H₂

### EXEMPLE : 4-(3-pyridinyl)-1H-imidazole

### Stade A₁ : N-[β,β-diméthoxy-2-(3-pyridinyl)éthyl]-formamide

On ajoute à 60°C 100 ml de formamide à la suspension préparée ci-dessus. On obtient ainsi une suspension que l'on utilise telle quelle dans le stade suivant. On obtient après séparation et purification le produit recherché.

### Stade A_{2 :} N-[β,β-diméthoxy-2-(3-pyridinyl)éthyl]-formamide

On chauffe pendant 5 heures à 80°C sous azote , 7,2 g de β,β-diméthoxy-3-pyridineéthanamine dans 15 ml de formamide. On maintient le mélange réactionnel sous agitation à 20°C pendant 16 heures. On obtient ainsi le produit recherché
(R⁺ 51 %) .
RMN CDCl₃ ppm
3,24(s) 3,26(s) 6H 2 CH30
3,55(d, s après éch.) CH₂-NHCO
3,75(d, s après éch.) CH₂-NHCO
5,80(m) H mobile
8,21(m) H mobile
7,32(m) H5
7,64(d, s après éch.) NH-CHO
8,00(d, s après éch.) NH-CHO
7,75 à 7,82(m) H₄
8,54(dd) 8,57(dd) H₆
8,68(m) 8,70(dd) H₂

### Stade B : 4-(3-pyridinyl)-1H-imidazole

On chauffe à 125°C en distillant le méthanol la suspension obtenue au stade A. On maintient 16 heures sous agitation. On obtient une suspension que l'on refroidit à 80°C et ajoute 400 ml d'eau déminéralisée. On ajoute à 80°C 130,3 g d'acide oxalique. On refroidit à 60°C. On agite 1 heure à 60°C, refroidit à 20°C, agite 1 heure à 20°C, refroidit à 0°C, agite 16 heures à 0°C essore et lave. On obtient ainsi l'oxalate du produit recherché. On obtient le 4-(3-pyridinyl)-H-imidazole par action d'une solution de potasse.
RMN CDCl₃ ppm
7,38 (dd, J=5 et 8) H₅
7,78(sl) 7,80(sl) H₂' H₅'
8,12(dl, J=8) H₄
8,41(dd,J=2 et 5) H₆
9,03(sl) H₂
12,36(m large)H mobile

1 heure à 60°C, refroidit à 20°C, agite 1 heure à 20°C, refroidit à 0°C, agite 16 heures à 0°C essore et lave. On obtient ainsi l'oxalate du produit recherché. On obtient le 4-(3-pyridinyl)-H-imidazole par action d'une solution de potasse.
RMN CDCl₃ ppm
7,38 (dd, J=5 et 8) H₅
7,78(sl) 7,80(sl) H₂' H₅'
8,12(dl, J=8) H₄
8,41(dd,J=2 et 5) H₆
9,03(sl) H₂
12,36(m large)H mobile

## Revendications

1. Procédé de préparation de composés de formule (I) 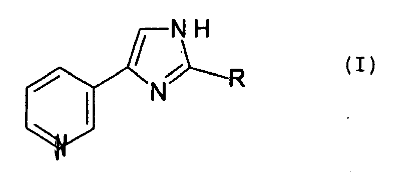 dans laquelle R représente un atome d'hydrogène ou un radical alkyle renfermant jusqu'à 8 atomes de carbone, **caractérisé en ce que** l'on soumet un composé de formule (A) : 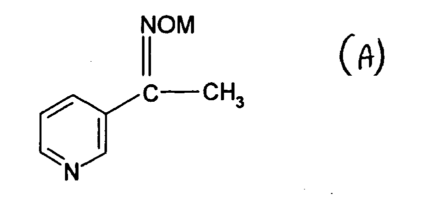 OM représentant un radical hydroxyle bloqué sous forme d'un bon groupe partant, à l'action d'un alcool et d'un alcoolate alcalin, alc₁OH/alC₂OL, alc₁ et alc₂, identiques ou différents, représentant un radical alkyle renfermant jusqu'à 4 atomes de carbone et L représentant un atome de sodium ou de potassium, pour obtenir la 3-(2H-azirin-3-yl)pyridine de formule B : 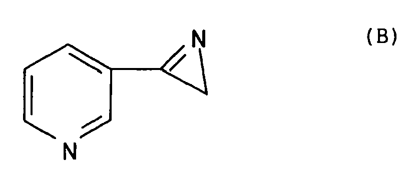 que l'on soumet, sans l'isoler du milieu réactionnel, à l'action d'un acide choisi parmi l'acide oxalique et l'acide formique, pour obtenir le composé de formule (II) correspondant : 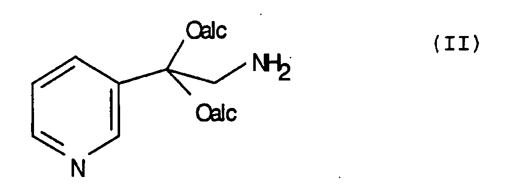 dans laquelle alc est un alkyle renfermant de 1 à 4 atomes de carbone, que l'on soumet, sans l'isoler du milieu réactionnel, à l'action d'un composé de formule (III) :
RCONH₂ (III)
dans laquelle R conserve sa signification précédente, pour obtenir le composé de formule (IV): 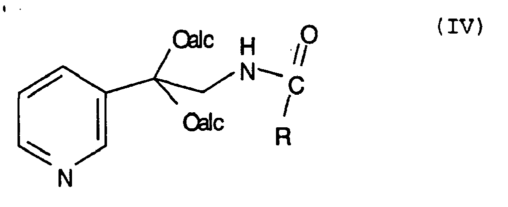 dans laquelle R conserve sa signification précédente, que l'on soumet à une cyclisation par chauffage pour obtenir le produit de formule (I) recherché.

2. Procédé selon la revendication 1, **caractérisé en ce que** l'on utilise un composé de formule III dans laquelle R représente un atome d'hydrogène.

3. Procédé selon la revendication 1 ou 2, **caractérisé en ce que** alc₁ et alc₂ représentent le même radical alkyle.

4. Procédé selon la revendication 1, 2 ou 3, **caractérisé en ce que** l'on opère sans isolement du produit intermédiaire de formule (IV).

5. Procédé de préparation selon l'une quelconque des revendications 1 à 4, **caractérisé en ce que** l'on soumet la O-[(4-méthylphényl) sulfonyl] oxime de 1-(3-pyridinyl)-éthanone à l'action du méthanol et du méthylate de sodium pour obtenir la 3-(2H-azirin-3-yl)-pyridine que l'on soumet, sans l'isoler du milieu réactionnel, à l'action de l'acide oxalique pour obtenir la β,β-diméthoxy-2-(3-pyridinyl)éthyl]amine que l'on soumet, sans l'isoler du milieu réactionnel, à l'action du formamide pour obtenir le N-[β,β-diméthoxy-2-(3-pyridinyl)éthyl]-formamide que l'on soumet, sans l'isoler du milieu réactionnel, à une cyclisation par chauffage pour obtenir le 4-(3-pyridinyl)-1H-imidazole.

## Claims

1. Process for preparing compounds of formula (I) 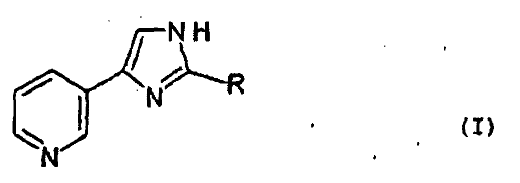 in which R represents a hydrogen atom or an alkyl radical containing up to 8 carbon atoms, **characterized in that** a compound of formula (A): 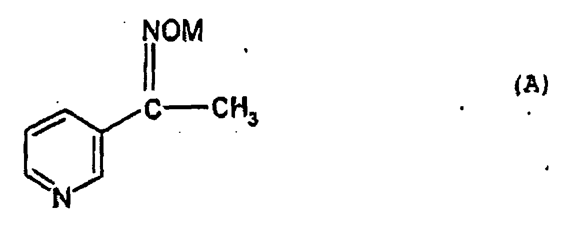 OM representing a hydroxyl radical that is blocked in the form of a good leaving group, is subjected to the action of an alcohol and of an alkali metal alcoholate, alc₁OH/alC₂OL, alc₁ and alc₂, which are identical or different, representing an alkyl radical containing up to 4 carbon atoms and L representing a sodium or potassium atom, in order to obtain 3-(2H-azirin-3-yl)pyridine of formula B: 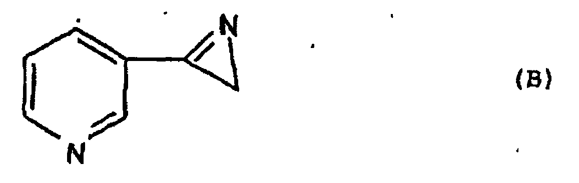 that is subjected, without isolating it from the reaction medium, to the action of an acid chosen from oxalic acid and formic acid, in order to obtain the corresponding compound of formula (II): 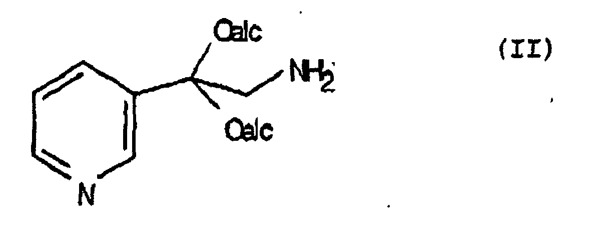 in which alc is an alkyl containing from 1 to 4 carbon atoms, which is subjected, without isolating it from the reaction medium, to the action of a compound of formula (III):
RCONH₂ (III)
in which R retains its previous meaning, in order to obtain the compound of formula (IV): 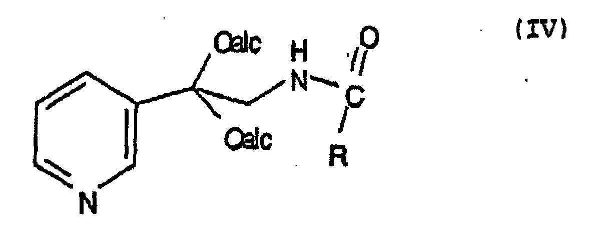 in which R retains its previous meaning, which is subjected to a cyclization by heating in order to obtain the desired product of formula (I).

2. Process according to Claim 1, **characterized in that** a compound of formula (III) is used in which R represents a hydrogen atom.

3. Process according to Claim 1 or 2, **characterized in that** alc₁ and alc₂ represent the same alkyl radical.

4. Process according to Claim 1, 2 or 3, **characterized in that** it is carried out without isolating the intermediate product of formula (IV).

5. Preparation process according to any one of Claims 1 to 4, **characterized in that** 1-(3-pyridinyl)ethanone O-[(4-methylphenyl)sulphonyl]oxime is subjected to the action of methanol and of sodium methylate in order to obtain 3-(2H-azirin-3-yl)pyridine which is subjected, without isolating it from the reaction medium, to the action of oxalic acid in order to obtain β,β-dimethoxy-2-(3-pyridinyl)ethyl]amine that is subjected, without isolating it from the reaction medium, to the action of formamide in order to obtain N-[β,β-dimethoxy-2-(3-pyridiinyl)ethyl]formamide that is subjected, without isolating it from the reaction medium, to a cyclization by heating in order to obtain 4-(3-pyridinyl)-1H-imidazole.

## Patentansprüche

1. Verfahren zur Herstellung von Verbindungen der Formel (I) 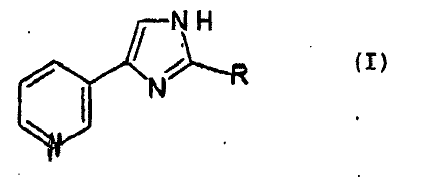 worin R für ein Wasserstoffatom oder einen Alkylrest mit bis zu 8 Kohlenstoffatomen steht, **dadurch gekennzeichnet, daß** man eine Verbindung der Formel (A) : 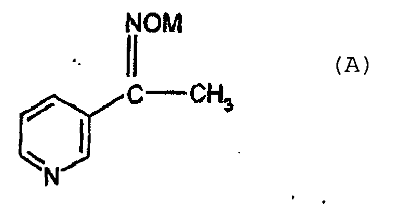 wobei OM für einen blockierten Hydroxylrest in Form einer guten Abgangsgruppe steht, der Einwirkung eines Alkohols und eines Alkalimetallalkoholats, Alk₁OH/Alk₂OL, wobei Alk₁ und Alk₂ gleich oder verschieden sind und für einen Alkylrest mit bis zu 4 Kohlenstoffatomen stehen und L für ein Natrium- oder Kaliumatom steht, unterwirft, was ein 3-(2H-Azirin-3-yl)pyridin der Formel B: 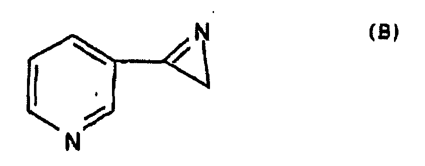 ergibt, welches man ohne Isolierung aus dem Reaktionsmedium der Einwirkung einer unter Oxalsäure und Ameisensäure ausgewählten Säure unterwirft, was die entsprechende Verbindung der Formel (II): 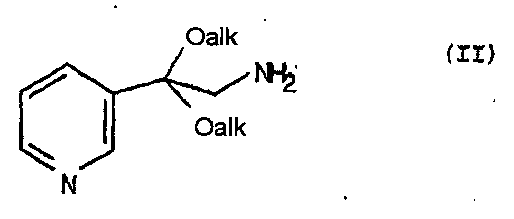 worin Alk für ein Alkyl mit 1 bis 4 Kohlenstoffatomen steht, ergibt, welches man ohne Isolierung aus dem Reaktionsmedium der Einwirkung einer Verbindung der Formel (III):
RCONH₂ (III)
worin R die oben angegebene Bedeutung behält, unterwirft, was die Verbindung der Formel (IV): 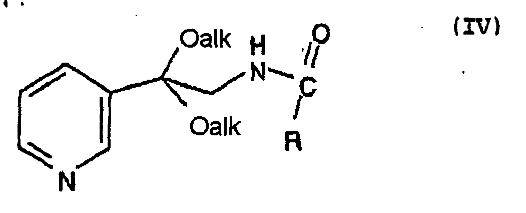 worin R die oben angegebene Bedeutung behält, ergibt, welche man einer Cyclisierung durch Erhitzen unterwirft, was das gewünschte Produkt der Formel (I) ergibt.

2. Verfahren nach Anspruch 1, **dadurch gekennzeichnet, daß** man eine Verbindung der Formel III verwendet, in der R für ein Wasserstoffatom steht.

3. Verfahren nach Anspruch 1 oder 2, **dadurch gekennzeichnet, daß** Alk₁ und Alk₂ für den gleichen Alkylrest stehen.

4. Verfahren nach Anspruch 1, 2 oder 3, **dadurch gekennzeichnet, daß** man ohne Isolierung des Zwischenprodukts der Formel (IV) arbeitet.

5. Verfahren nach einem der Ansprüche 1 bis 4, **dadurch gekennzeichnet, daß** man 1-(3-Pyridinyl)-ethanon-O-[4-(methylphenyl)sulfonyl]oxim der Einwirkung von Methanol und Natriummethylat unterwirft, was 3-(2H-Azirin-3-yl)pyridin ergibt, welches man ohne Isolierung aus dem Reaktionsmedium der Einwirkung von Oxalsäure unterwirft, was β,β-Dimethoxy-2-(3-pyridinyl)ethyl]amin ergibt, welches man ohne Isolierung aus dem Reaktionsmedium der Einwirkung von Formamid unterwirft, was N-[β,β-Dimethoxy-2-(3-pyridinyl)ethyl]-formamid ergibt, welches man ohne Isolierung aus dem Reaktionsmedium einer Cyclisierung durch Erhitzen unterwirft, was 4-(3-Pyridinyl)-1H-imidazol ergibt.
